# EUROPÄISCHE PATENTSCHRIFT

(11) **EP 1 355 147 B1**
(45) Veröffentlichungstag und Bekanntmachung des Hinweises auf die Patenterteilung: **12.10.2005**
(21) Anmeldenummer: 03090084.9
(22) Anmeldetag: 27.03.2003
(51) Int. Cl.: G01N 33/14, G01N 21/35, G01N 1/22

(54) **Sonde zur Alkoholmessung in Flüssigkeiten**
Probe for measuring the alcohol content in liquids
Sonde pour mesurer la teneur en alcool de liquides

(30) Priorität: 15.04.2002 DE 10216653
(43) Veröffentlichungstag der Anmeldung: 22.10.2003
(73) Patentinhaber: Biotechnologie Kempe GmbH, 14195 Berlin (DE)
(72) Erfinder: Kempe, Eberhard, Dipl.-Ing., 10719 Berlin (DE)
(74) Vertreter: Lüke, Dierck-Wilm

(56) Entgegenhaltungen:
- EP-A- 0 494 734
- EP-A- 0 795 744
- WO-A-93/17324
- DE-A- 10 025 686
- DE-U- 20 100 557
- US-A- 4 041 932
- US-A- 4 228 192

## Beschreibung

### Gebiet der Erfindung.

Die Erfindung betrifft eine Sonde zur quantitativen Messung flüchtiger Bestandteile einer Flüssigkeit mit einem Messgasraum, wobei der Messgasraum von der Flüssigkeit durch eine für den flüchtigen Bestandteil durchlässige, jedoch flüssigkeitsundurchlässige Permeationsmembran abgetrennt ist, und mit einem im Messgasraum angeordneten selektiven Gasdetektionssystem für einen definierten flüchtigen Bestandteil.

### Hintergrund und Stand der Technik.

Zur Überwachung, Steuerung und/oder Regelung von bestimmten chemischen, biotechnologischen, nahrungsmitteltechnischen oder pharmazeutischen Prozessen ist es notwendig den Alkoholgehalt einer Lösung oder Suspension zu bestimmen und zu überwachen, on-line bzw. in-line oder off-line. Weiterhin ist es regelmäßig wünschenswert, weitere flüchtige Bestandteile der Lösung oder Suspension, wie CO₂, gleichzeitig zu bestimmen und zu überwachen. Ein Beispiel eines wirtschaftlich wichtigen Prozesses ist die Bierbrauerei.

Flüchtige Bestandteile einer Flüssigkeit können neben C1 bis C6-Alkyl-Monoalkoholen C1 bis C8-Kohlenwasserstoffe, C1 bis C6-Alkylaldehyde, C1 bis C6-Alkyl-Ketone, C1 bis C6-Alkyl-Carboxylsäuren, CO₂ und O₂ sein. Im Bereich der Bierbrautechnik sind insbesondere die C1 bis C3-Alkyl-Monoalkohole, insbesondere Ethanol, CO₂ und/oder O₂ zu bestimmen und zu überwachen.

Beispielsweise aus den Literaturstellen EP 0 174 417 B1 und DE-199 59 271 A1 sind Sonden bekannt, mittels welcher on-line bzw. in-line der Alkoholgehalt einer Flüssigkeit bestimmbar ist. Im Rahmen der insofern bekannten Maßnahmen erfolgt die Alkoholbestimmung mittels eines Festkörperdetektors. Ein Festkörperdetektor umfaßt typischerweise ein Halbleiterelement, beispielsweise auf Basis Zinnoxid, an dessen Oberfläche Alkohol umgesetzt wird, wodurch ein elektrisches Signal entsteht, welches einer Auswerteelektronik zugeführt wird. Die insofern bekannte Technologie hat sich gut bewährt. Es besteht allerdings ein Bedarf für die Bestimmung hoher Alkoholgehalte, i.e. im Bereich von 2 Vol-% (bezogen auf die Flüssigkeit) bis zu 20 Vol-%. Bei solch hohen Alkoholkonzentration sind Messungen mit Festkörperdetektoren jedoch ungenau bzw. nicht möglich.

Aus der Literaturstelle DE 100 25 686 A1 ist es bekannt, in der Bierbrautechnik gleichzeitig den Alkoholgehalt und die Brechzahl einer Flüssigkeit zu bestimmen. Im Rahmen dieser Maßnahmen wird ebenfalls ein Zinnoxid-Detektor eingesetzt mit dem vorstehenden Nachteil.

Aus der Literaturstelle DE 20100557 U1 ist eine Sonde gemäß dem Oberbegriff des Patentanspruchs 1 bekannt. Aus der Literaturstelle US-A-4,041,932 ist es grundsätzlich bekannt, ein Gasdetektionssystem in einem Messgasraum anzuordnen.

Technisches Problem der Erfindung.

Der Erfindung liegt das technische Problem zu Grunde, eine Sonde anzugeben, mittels welcher sich zuverlässig hohe Alkoholkonzentrationen in Flüssigkeiten bestimmen lassen.

Grundzüge der Erfindung und bevorzugte Ausführungsbeispiele.

Zur Lösung dieses technischen Problems lehrt die Erfindung, daß das Gasdetektionssystem im Messgasraum angeordnet ist, und dass das IR-Filter ein Transmissionsfenster im Bereich einer alkoholspezifischen Absorptionsbande und einer Wellenlänge von 1,0 bis 3,0 µm aufweist.

Der Begriff des IR-empfindlichen Lichtsensors umfaßt neben Photosensoren auch thermische Sensoren, beispielsweise pyroelektrische Sensoren. Eine IR-Lichtquelle strahlt IR-Licht im Bereich des Transmissionsfensters aus. Als Transmissionsfenster ist bezeichnet, die Eigenschaft eines Bauelementes, um eine definierte Mittenwellenlänge nur einen durch die Halbwertsbreite (bezogen auf die maximale Transmission) definierbaren Wellenlängenbereich durchzulassen. Wellenlängenangabe beziehen sich im Folgenden auf die Mittenwellenlänge. Transmissionfenster überlappen nicht, wenn die Halbwertsbreiten nicht überlappen.

Die Erfindung beruht zunächst auf der Erkenntnis, daß hohe Alkoholgehalte auch mit den üblichen Permeationsmembranen meßtechnisch beherrschbar sind. Hieran anschließend wurde erkannt, daß mittels IR-Absorptionsmessung ein lineares bzw. leicht linearisierbares (beispielsweise durch Kalibrierung mit einer. Übertragungsfunktion erhalten durch Messung einer Referenzlösung oder mehrerer Referenzlösungen) Signal erhalten wird, und zwar bei Konzentrationen bis zu 20 Vol.-% und höher in der Flüssigkeit. Von besondere Bedeutung ist hierbei, daß nicht nur eine Erhöhung eines Trägergasstromes durch den Messgasraum entbehrlich ist (mit dieser Maßnahme könnte auch mittels Halbleiterdetektoren zu höheren Alkoholgehalten hin gemessen werden), vielmehr kann sogar ganz ohne Trägergasstrom gearbeitet werden. Dies erlaubt es, mobile, tragbare Sonden zu schaffen, da die Mitführung einer Trägergasquelle entfällt. Vor einer Messung bzw. zwischen zwei Messungen muß lediglich eine Spülung des Messgasraumes, beispielsweise mittels der Umgebungsluft, erfolgen.

Das Transmissionsfenster liegt zweckmäßigerweise bei einer Wellenlänge von 1,0 bis 3,0 µm, vorzugsweise 1,5 bis 2,5 µm, insbesondere 2,0 bis 2,2 µm.

Das IR-Filter kann grundsätzlich an verschiedenen Orten angeordnet sein. Ist das Filter unmittelbar bei der IR-Lichtquelle angeordnet, so durchflutet ausschließlich Licht der Wellenlängen des Transmissionsfenster den Messgasraum oder Teile hiervon. Dann braucht der Lichtsensor nicht wellenlängenselektiv zu arbeiten. Bevorzugt ist es jedoch, wenn das IR-Filter unmittelbar dem Lichtsensor vorgeschaltet ist. Dann durchflutet Licht mit Wellenlängen des gesamten IR-Bereiches den Messgasraum bzw. Teile hiervon, während der Verbund aus Lichtsensor und IR-Filter einen wellenlängenselektiven Lichtsensor bildet. In jedem Fall empfiehlt es sich, daß der Lichtsensor gegen Fremdlicht abgeschattet bzw. abgeschirmt ist.

Für stationäre Anwendungen, beispielsweise für die on-line bzw. in-line Messung in einer strömenden Flüssigkeit, wobei die Permeationsmembran in die Flüssigkeit eintaucht, ist es bevorzugt, wenn der Messgasraum von einem vorzugsweise kontinuierlichen Trägergasstrom durchströmt wird, also die Alkoholmoleküle konvektiv durch den Messgasraum transportiert werden. Als Trägergas kommen Luft, Stickstoff etc. in Frage. Mit der Einstellung eines definierten Trägergasstromes wird eine sehr hohe Genauigkeit erreicht. Dabei kann die Flüssigkeit an der Permeationsmembran vorbeiströmen (in-line-Messungen) oder ruhen (Labormessung einer Probe)

Für mobile bzw. tragbare Ausführungsformen der Erfindung ist dagegen bevorzugt, wenn trägergaslos gearbeitet wird. Es erfolgt lediglich vor einer Messung eine Spülung des Messgasraumes mit beispielsweise Luft. Hierzu kann eine kleine Pumpe vorgesehen sein, welche an einen Spülgaseinlaß angeschlossen ist. Zweckmäßigerweise ist auch ein Spülgasauslaß vorgesehen. Nach der Spülung werden Spülgaseinlaß und Spülgasauslaß verschlossen. Die Permeationsmembran wird dann in eine entnommene Flüssigkeitsprobe eingetaucht, und zwar solange, bis sich ein konstanter Meßwert ergibt, i.e. sich ein Gleichgewicht zwischen Alkoholgehalt der Probe und Alkoholgehalt der Messgasraumes eingestellt hat. Der Transport der Alkoholmoleküle in der Gasphase erfolgt also rein diffusiv.

In einer Weiterbildung der Erfindung von selbstständiger bedeutung sind ein zweiter Lichtsensor und ein zweites IR-Filter, vorzugsweise dem zweiten Lichtsensor unmittelbar vorgeschaltet, im Messgasraum eingerichtet, wobei das zweite IR-Filter ein Transmissionsfenster im Bereich einer CO₂-spezifischen Absorptionsbande liegt, und wobei die Transmissionsfenster des ersten IR-Filters und des zweiten IR-Filters sich nicht überlappen. In dieser Ausführungform läßt sich mit einer einzigen Sonde neben Alkohol gleichzeitig CO₂ messen. Im Falle des trägergaslosen Arbeitens und des Spülens mit Luft erfolgt selbstverständlich eine Nullmessung des CO₂, deren Wert von dem dann gemessenen Wert zwecks Bestimmung des CO₂ Gehaltes der Flüssigkeit abgezogen wird. Anstelle von CO₂ oder zusätzlich hierzu kann die Sonde für die gleichzeitige Messung weiterer flüchtiger Bestandteile eingerichtet sein durch Hinzufügung von Lichtsensoren und Filtern mit entsprechenden Transmissionsfenstern.

Im Falle der CO₂ Messung ist es bevorzugt, wenn das Transmissionsfenster des zweiten IR-Filter bei einer Wellenlänge von 4,0 bis 5,0 µm, insbesondere 4,1 bis 4,5 µm, liegt.

Die Messung weiterer Komponenten, insbesondere CO₂ neben Alkohol, kann unterschiedlich vorgesehen sein. Der Messgasraum kann aufweisen: i) einen Permeationsgasraum, ii) eine erste IR-Messkuvette mit erster IR-Lichtquelle, erstem IR-Filter und erstem Lichtsensor, und iii) eine zweite IR-Messkuvette mit zweiter IR-Lichtquelle, zweitem IR-Filter und zweitem Lichtsensor, wobei der Permeationsgasraum, die erste IR-Messkuvette und die zweite IR-Messkuvette gasdiffusiv oder konvektiv miteinander verbunden sind, und wobei die Transmissionsfenster des ersten IR-Filter und des zweiten IR-Filter einander nicht überlappen und in einem Bereich einer alkoholspezifischen sowie einer CO₂-spezifischen Absorptionsbande liegen. Alternativ kann der Messgasraum aufweisen: i) einen Permeationsgasraum, und ii) eine einzige IR-Messkuvette mit einer IR-Lichtquelle, erstem IR-Filter, zweitem IR-Filter und zumindest einem Lichtsensor, wobei der Permeationsgasraum und die IR-Messkuvette gasdiffusiv oder konvektiv miteinander verbunden sind, und wobei die Transmissionsfenster des ersten IR-Filter und des zweiten IR-Filter einander nicht überlappen und in einem Bereich einer alkoholspezifischen sowie einer CO₂-spezifischen Absorptionsbande liegen. In letzterem Fall wird eine besondere bauliche Einfachheit erreicht.

Eine weitere bauliche Vereinfachung sowie Reduzierung der Abmessungen und des Gewichtes wird erreicht, wenn die Sonde einen Sondenfinger mit einem durch die Permeationsmembran von der Flüssigkeit abgetrennten Sondenfingergasraum und einen den Sondenfinger tragenden Sondenkörper aufweist, wobei die zumindest eine IR-Messkuvette im Sondenkörper angeordnet und diffusiv oder konvektiv mit dem Sondenfingergasraum verbunden ist. Alternativ kann die Sonde einen Sondenfinger mit einem durch die Permeationsmembran von der Flüssigkeit abgetrennten Sondenfingergasraum und einen den Sondenfinger tragenden Sondenkörper mit einem Sondenkörpergasraum sowie eine von dem Sondenkörper getragenen zumindest eine IR-Messkuvette aufweisen, wobei der Sondenfingergasraum, der Sondenkörpergasraum und die IR-Kuvette diffusiv oder konvektiv miteinander verbunden sind.

Im Rahmen des Messgasraumes können weitere Detektoren für verschiedenste flüchtige Bestandteile eingerichtet sein. So ist es auch möglich, zur Messung in den niedrigen Alkoholkonzentrationsbereichen (bis zu 2 Vol.-%) im Messgasraum, insbesondere im Sondenkörpergasraum, ein alkoholspezifischer Festkörpergassensor einzurichten.

Im folgenden wird die Erfindung anhand der folgenden, lediglich ein Ausführungsbeispiel darstellenden Figuren näher erläutert. Es zeigt:
- Fig. 1:: eine erfindungsgemäße Sonde zur kombinierten Messung von Alkohol und CO₂ und
- Fig. 2:: eine erfindungsgemäße Sonde in tragbarer Variante.

In der Figur erkennt man eione Sonde 1 zur quantitativen Messung flüchtiger Bestandteile einer Flüssigkeit 2, welche ein Rohr 4 durchströmt mit einem Messgasraum 3. Der Messgasraum 3 ist von der Flüssigkeit 2 durch eine für den flüchtigen Bestandteil durchlässige, jedoch flüssigkeitsundurchlässige Permeationsmembran 5 abgetrennt. Es ist ein Gasdetektionssystem eingerichtet, welches eine IR-Lichtquelle 6, zwei IR-Filter 7, 7' sowie zwei IR-empfindlichen Lichtsensoren 8, 8' aufweist, wobei Licht durch die IR-Filter 7, 7'auf die Lichtsensoren 8, 8' fällt. Das IR-Filter 7 weist ein Transmissionsfenster im Bereich einer alkoholspezifischen Absorptionsbande auf, nämlich bei 2,09 µm. Das IR-Filter 7' weist ein Transmissionsfenster im Bereich einer CO₂-spezifischen Absorptionsbande auf, nämlich bei 4,3 µm. Die Lichtsensoren 8, 8' sind pyroelektrische Detektoren und beispielsweise erhältlich von der Firma Laser Components GmbH, Deutschland (beispielsweise aus der LIE Serie). Die IR-Filter 7, 7' sind integriert bzw. den Lichtsensoren 8, 8' unmittelbar vorgeschaltet. Die Detektoren sind Doppeldetektoren, i.e. umfassen jeweils zwei Sensoren, wobei einer durch ein IR-Filter 7, 7' abgeschattet ist.

Im Ausführungsbeispiel, einer Vorrichtung zur in-line Messung, ist der Messgasraum 3 von einem kontinuierlichen Trägergasstrom durchströmt, wozu ein Trägergaseinlaß 15 und ein Trägergasauslaß 16 vorgesehen sind.

Im Einzelnen weist der Messgasraum 3 auf: i) einen Permeationsgasraum 9, und ii) eine einzige IR-Messkuvette 10 mit einer IR-Lichtquelle 6, erstem IR-Filter 7, zweitem IR-Filter 7' und zumindest zwei Lichtsensoren 8, 8', wobei der Permeationsgasraum 9 und die IR-Messkuvette 10 mittels des Trägergasstromes konvektiv miteinander verbunden sind. Es ist ein in die Flüssigkeit 2 eintauchender Sondenfinger 11 mit einem durch die Permeationsmembran 5 von der Flüssigkeit 2 abgetrennten Sondenfingergasraum 12 und einen den Sondenfinger 11 tragenden Sondenkörper 13 eingerichtet, wobei die IR-Messkuvette 10 im Sondenkörper 13 angeordnet ist.

Weiterhin erkennt man an die Lichtsensoren 8, 8' angeschlossene Verstärker 17, 18, deren Ausgangssignal einer Auswerteeinheit 19 zugeführt wird. In der Auswerteeinheit 19 werden die Signale unter Berücksichtigung von abgespeicherten Eichkurven in Konzentrationen, bezogen auf die Konzentrationen in der Flüssigkeit, umgerechnet und in einer Anzeige- und Bedieneinheit 20 angezeigt. An die Auswerteinheit 19 ist weiterhin ein Temperatursensor 21 angeschlossen, dessen Messwert der Flüssigkeitstemperatur bei der Berechnung der Konzentrationen berücksichtigt wird.

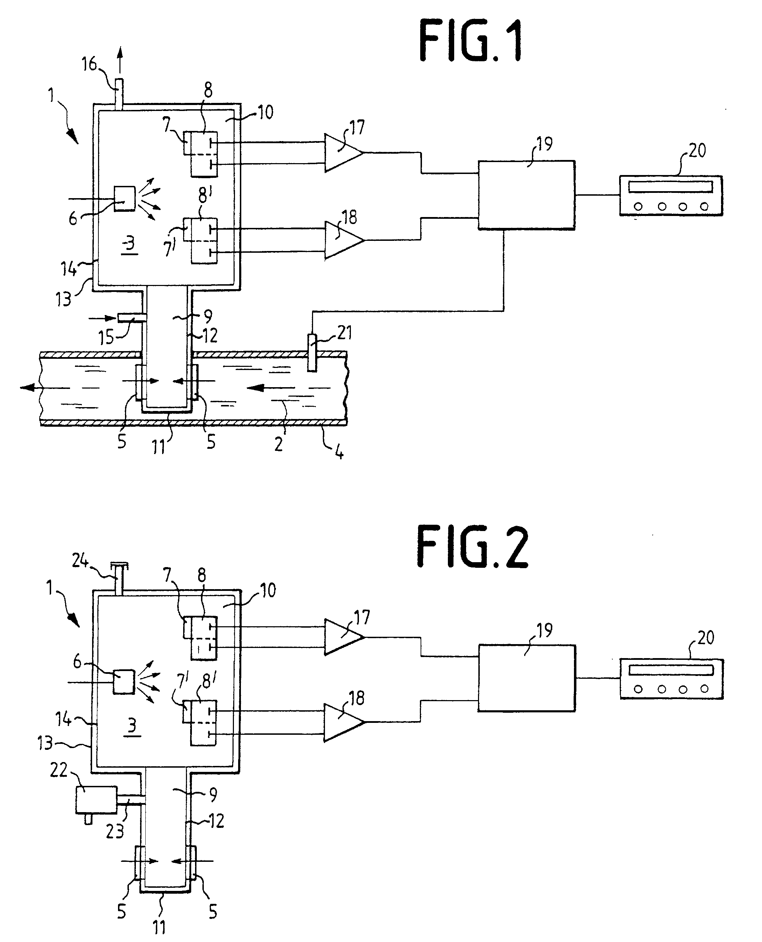

In der Figur 2 ist eine tragbare Variante einer erfindungsgemäßen Sonde 1 dargestellt. Die wesentlichen Elemente entsprechen jener der Figur1, auf welche diesbezüglich verwiesen wird. Abweichend ist jedoch kein Trägergaseinlaß 15 und Trägergasauslaß 16 eingerichtet. Stattdessen ist eine Spülluftpumpe 22 eingerichtet, deren Druckseite über einen Spüllufteinlaß 23 mit dem Messgasraum 3 verbunden ist. Die Saugseite steht mit der Umgebungsluft in Verbindung. Weiterhin ist ein Spülluftauslaß 24 vorgesehen, welcher verschließbar ist. Spüllufteinlaß 23 und Spülluftauslaß 24 können baulich identisch mit dem Trägergaseinlaß 15 und Trägergasauslaß 16 ausgebildet sein, so daß sich die Sonde 1 in beiden Varianten, in-line bzw. im Laborbetrieb oder mobil, betreiben läßt.

## Patentansprüche

1. Sonde (1) zur quantitativen Messung flüchtiger Bestandteile einer Flüssigkeit (2) mit einem Messgasraum (3), wobei der Messgasraum (3) von der Flüssigkeit (4) durch eine für den flüchtigen Bestandteil durchlässige, jedoch flüssigkeitsundurchlässige Permeationsmembran (5) abgetrennt ist, und mit einem selektiven Gasdetektionssystem für einen definierten flüchtigen Bestandteil,
wobei das Gasdetektionssystem eine IR-Lichtquelle (6), ein IR-Filter (7), (7)' sowie einen IR-empfindlichen Lichtsensor (8), (8)' aufweist, wobei Licht durch das IR-Filter auf den Lichtsensor (8), (8)' fällt
**dadurch gekennzeichnet,**
**dass** das Gasdetektionssystem im Messgasraum (3) angeordnet ist, und
**dass** das IR-Filter (7), (7)' ein Transmissionsfenster im Bereich einer alkoholspezifischen Absorptionsbande und einer Wellenlänge von 1,0 bis 3,0 µm aufweist.

2. Sonde (1) nach Anspruch 1, **dadurch gekennzeichnet, dass** das Transmissionsfenster bei einer Wellenlänge von 1,5 bis 2,5 µm, insbesondere 2,0 bis 2,2 µm, liegt.

3. Sonde (1) nach Anspruch 1 oder 2, **dadurch** gekennziechnet, dass das IR-Filter (7), (7)' unmittelbar dem Lichtsensor (8), (8)' vorgeschaltet ist.

4. Sonde (1) nach einem der Ansprüche 1 bis 3, **dadurch gekennzeichnet, dass** der Messgasraum (3) von einem kontinuierlichen Trägergasstrom durchströmt wird.

5. Sonde (1) nach einem der Ansprüche 1 bis 4, **dadurch gekennzeichnet, dass** ein zweiter Lichtsensor (8)' und ein zweites IR-Filter (7)', vorzugsweise dem zweiten Lichtsensor (8)' unmittelbar vorgeschaltet, im Messgasraum (3) eingerichtet ist, wobei das zweite IR-Filter (7)' ein Transmissionsfenster im Bereich einer CO₂-spezifischen Absorptionsbande liegt, und wobei die Transmissionsfenster des ersten IR-Filters (7) und des zweiten IR-Filters (7)' sich nicht überlappen.

6. Sonde nach Anspruch 5, **dadurch gekennzeichnet, dass** das Transmissionsfenster des zweiten IR-Filters (7)' bei einer Wellenlänge von 3,0 bis 5,0 µm, vorzugsweise 4,0 bis 5,0 µm, insbesondere 4,1 bis 4,5 µm, liegt.

7. Sonde (1) nach einem der Ansprüche 1 bis 6, **dadurch gekennzeichnet, dass** der Messgasraum (3) aufweist: i) einen Permeationsgasraum (9), ii) eine erste IR-Messkuvette (10) mit erster IR-Lichtquelle (6), erstem IR-Filter (7) und erstem Lichtsensor (8), und iii) eine zweite IR-Messkuvette mit zweiter IR-Lichtquelle, zweitem IR-Filter und zweitem Lichtsensor, wobei der Permeationsgasraum (9), die erste IR-Messkuvette (10) und die zweite IR-Messkuvette gasdiffusiv oder konvektiv miteinander verbunden sind, und wobei die Transmissionsfenster des ersten IR-Filter (7) und des zweiten IR-Filter (7) einander nicht überlappen und in einem Bereich einer alkoholspezifischen sowie einer CO₂-spezifischen Absorptionsbande liegen.

8. Sonde (1) nach einem der Ansprüche 1 bis 6, **dadurch gekennzeichnet, dass** der Messgasraum (3) aufweist: i) einen Permeationsgasraum (9), und ii) eine einzige IR-Messkuvette (10) mit einer IR-Lichtquelle (6), erstem IR-Filter (7), zweitem IR-Filter (7)' und zumindest einem Lichtsensor (8), (8)', wobei der Permeationsgasraum (9) und die IR-Messkuvette (10) gasdiffusiv oder konvektiv miteinander verbunden sind, und wobei die Transmissionsfenster des ersten IR-Filter (7) und des zweiten IR-Filter (7)' einander nicht überlappen und in einem Bereich einer alkoholspezifischen sowie einer CO₂-spezifischen Absorptionsbande liegen.

9. Sonde (1) nach einem der Ansprüche 1 bis 8, **dadurch gekennzeichnet, dass** die Sonde (1) einen Sondenfinger (11) mit einem durch die Permeationsmembran (5) von der Flüssigkeit (2) abgetrennten Sondenfingergasraum (12) und einen den Sondenfinger (11) tragenden Sondenkörper (13) aufweist, wobei die zumindest eine IR-Messkuvette (10) im Sondenkörper (13) angeordnet und diffusiv oder konvektiv mit dem Sondenfingergasraum (12) verbünden ist.

10. Sonde (1) nach einem der Ansprüche 1 bis 8, **dadurch gekennzeichnet, dass** die Sonde (1) einen Sondenfinger (11) mit einem durch die Permeationsmembran (5) von der Flüssigkeit (2) abgetrennten Sondenfingergasraum (12) und einen den Sondenfinger (11) tragenden Sondenkörper (13) mit einem Sondenkörpergasraum (14) sowie eine von dem Sondenkörper (13) getragenen zumindest eine IR-Messkuvette (10) aufweist, wobei der Sondenfingergasraum (12), der Sondenkörpergasraum (14) und die IR-Kuvette (10) diffusiv oder konvektiv miteinander verbunden sind.

11. Sonde (1) nach einem der Ansprüche 1 bis 10, **dadurch gekennzeichnet, dass** im Messgasraum (3), insbesondere im Sondenkörpergasraum (14), ein alkoholspezifischer Festkörpergassensor eingerichtet ist.

## Claims

1. A probe (1) for quantitatively measuring volatile components of a liquid (2), including a measurement gas space (3), said measurement gas space (3) being separated from the liquid (4) by a permeation membrane (5) permeable for the volatile component, however non-permeable for liquid, and a selective gas detection system for a defined volatile component,
the gas detection system including an IR light source (6), an IR filter (7), (7') and an IR-sensitive light sensor (8), (8') with light falling through the IR filter on the light sensor (8), (8'),
**characterized by**
that the gas detection system is arranged in the measurement gas space (3), and
that the IR filter (7), (7') has a transmission window in the range of an alcohol-specific absorption band and a wavelength from 1.0 to 3.0 µm.

2. A probe (1) according to claim 1, **characterized by** that the transmission window is arranged at a wavelength from 1.5 to 2.5 µm, in particular 2.0 to 2.2 µm.

3. A probe (1) according to claim 1 or 2, **characterized by** that the IR filter (7), (7') is arranged immediately in front of the light sensor (8), (8').

4. A probe (1) according to one of claims 1 to 3, **characterized by** that the measurement gas space (3) is passed by a continuous carrier gas flow.

5. A probe (1) according to one of claims 1 to 4, **characterized by** that a second light sensor (8') and a second IR filter (7'), preferably immediately in front of the second light sensor (8'), are provided in the measurement gas space (3), the second IR filter (7') having a transmission window in the range of a CO₂-specific absorption band, and the transmission windows of the first IR filter (7) and of the second IR filter (7') not overlapping each other.

6. A probe (1) according to claim 5, **characterized by** that the transmission window of the second IR filter (7') is arranged at a wavelength from 3.0 to 5.0 µm, preferably from 4.0 to 5.0 µm, in particular from 4.1 to 4.5 µm.

7. A probe (1) according to one of claims 1 to 6, **characterized by** that the measurement gas space (3) comprises: i) a permeation gas space (9), ii) a first IR measurement cuvette (10) with a first IR light source (6), a first IR filter (7) and a first light sensor (8), and iii) a second IR measurement cuvette with a second IR light source, a second IR filter and a second light sensor, the permeation gas space (9), the first IR measurement cuvette (10) and the second IR measurement cuvette being connected to each other in a gas-diffusive or convective manner, and the transmission windows of the first IR filter (7) and of the second IR filter (7') not overlapping each other and being provided in a range of an alcohol-specific and a CO₂-specific absorption band.

8. A probe (1) according to one of claims 1 to 6, **characterized by** that the measurement gas space (3) comprises: i) a permeation gas space (9), and ii) a single IR measurement cuvette (10) with an IR light source (6), a first IR filter (7), a second IR filter (7') and at least one light sensor (8), (8'), the permeation gas space (9) and the IR measurement cuvette (10) being connected to each other in a gas-diffusive or convective manner, and the transmission windows of the first IR filter (7) and of the second IR filter (7') not overlapping each other and being provided in a range of an alcohol-specific and a CO₂-specific absorption band.

9. A probe (1) according to one of claims 1 to 8, **characterized by** that the probe (1) comprises a probe finger (11) with a probe finger gas space (12) separated from the liquid (2) by the permeation membrane (5) and a probe body (13) carrying the probe finger (11), the at least one IR measurement cuvette (10) being arranged in the probe body (13) and being connected with the probe finger gas space (12) in a diffusive or convective manner.

10. A probe (1) according to one of claims 1 to 8, **characterized by** that the probe (1) comprises a probe finger (11) with a probe finger gas space (12) separated from the liquid (2) by the permeation membrane (5) and a probe body (13) carrying the probe finger (11) with a probe body gas space (14) and at least one IR measurement cuvette (10) carried by the probe body (13), the probe finger gas space (12), the probe body gas space (14) and the IR cuvette (10) being connected to each other in a diffusive or convective manner.

11. A probe (1) according to one of claims 1 to 10, **characterized by** that in the measurement gas space (3), in particular in the probe body gas space (14), an alcohol-specific solid-state gas sensor is provided.

## Revendications

1. Sonde (1) pour mesurer quantitativement des composants volatils d'un liquide (2), comprenant un espace du gaz à mesurer (3), cet espace du gaz à mesurer (3) étant séparé du liquide (4) par une membrane de perméation (5) perméable au composant volatil, mais non-perméable au liquide, et un système de détection de gaz pour un composant volatil défini,
le système de détection de gaz comprenant une source de lumière (6), un filtre IR (7), (7') et un récepteur de lumière (8), (8') sensible à l'IR, la lumière tombant à travers des filtres IR (7), (7') sur les récepteurs de lumière (8), (8'),
**caractérisée en ce**
**que** le système de détection de gaz est positionné dans l'espace du gaz à mesurer (3), et
**que** le filtre IR (7), (7') comporte une fenêtre de transmission dans le domaine d'une bande d'absorption spécifique à l'alcool et une longueur d'onde de 1,0 à 3,0 µm.

2. Sonde (1) selon la revendication 1, **caractérisée en ce que** la fenêtre de transmission se trouve à une longueur d'onde de 1,5 à 2,5 µm, en particulier 2,0 à 2,2 µm.

3. Sonde (1) selon la revendication 1 ou 2, **caractérisée en ce que** le filtre IR (7), (7') est disposé immédiatement devant le récepteur de lumière (8), (8').

4. Sonde (1) selon une des revendications 1 à 3, **caractérisée en ce que** l'espace du gaz à mesurer (3) est passé par un courant continu de gaz porteur.

5. Sonde (1) selon une des revendications 1 à 4, **caractérisée en ce que** un deuxième récepteur de lumière (8') et un deuxième filtre IR (7'), de préférence disposé immédiatement devant le deuxième récepteur de lumière (8'), sont prévus dans l'espace du gaz à mesurer (3), le deuxième filtre IR (7') ayant une fenêtre de transmission dans le domaine d'une bande d'absorption spécifique à CO₂, et les fenêtres de transmission du premier filtre IR (7) et du deuxième filtre IR (7') ne se recouvrant pas.

6. Sonde (1) selon la revendication 5, **caractérisée en ce que** la fenêtre de transmission du deuxième filtre IR (7') se trouve à une longueur d'onde de 3,0 à 5,0 µm, de préférence 4,0 à 5,0 µm, en particulier 4,1 à 4,5 µm.

7. Sonde (1) selon une des revendications 1 à 6, **caractérisée en ce que** l'espace du gaz à mesurer (3) comporte: i) un espace du gaz de perméation (9), ii) une première cuvette de mesurage IR (10) avec une première source de lumière IR (6), un premier filtre IR (7) et un premier récepteur de lumière (8), et iii) une deuxième cuvette de mesurage IR avec une deuxième source de lumière IR, un deuxième filtre IR et un deuxième récepteur de lumière, l'espace du gaz de perméation (9), la première cuvette de mesurage IR (10) et la deuxième cuvette de mesurage IR étant raccordés l'un à l'autre en manière diffusive de gaz ou convective, et les fenêtres de transmission du premier filtre IR (7) et du deuxième filtre IR (7') ne se recouvrant pas et étant prévues dans un domaine de bande d'absorption spécifique à l'alcool et spécifique à CO₂.

8. Sonde (1) selon une des revendications 1 à 6, **caractérisée en ce que** l'espace du gaz à mesurer (3) comporte: i) un espace du gaz de perméation (9), et ii) une cuvette de mesurage IR (10) unique avec une source de lumière IR (6), un premier filtre IR (7), un deuxième filtre IR (7') et au moins une récepteur de lumière (8), (8'), l'espace du gaz de perméation (9) et la cuvette de mesurage IR (10) étant raccordés l'un à l'autre en manière diffusive de gaz ou convective, et les fenêtres de transmission du premier filtre IR (7) et du deuxième filtre IR (7') ne se recouvrant pas et étant prévues dans un domaine de bande d'absorption spécifique à l'alcool et spécifique à CO₂.

9. Sonde (1) selon une des revendications 1 à 8, **caractérisée en ce que** la sonde (1) comporte un doigt de sonde (11) avec un espace du gaz du doigt de sonde (12) séparé du liquide (2) par la membrane de perméation (5) et un corps de sonde (13) portant le doigt de sonde (11), au moins une cuvette de mesurage IR (10) étant prévue dans le corps de sonde (13) et étant raccordée en manière diffusive ou convective à l'espace du gaz du doigt de sonde (12).

10. Sonde (1) selon une des revendications 1 à 8, **caractérisée en ce que** la sonde (1) comporte un doigt de sonde (11) avec un espace du gaz du doigt de sonde (12) séparé du liquide (2) par la membrane de perméation (5) et un corps de sonde (13) portant le doigt de sonde (11) avec un espace du gaz du corps de sonde (14) et au moins une cuvette de mesurage IR (10) portée par le corps de sonde (13), l'espace du gaz du doigt de sonde (12), l'espace du gaz du corps de sonde (14) et la cuvette de mesurage IR (10) étant raccordés l'un à l'autre en manière diffusive ou convective.

11. Sonde (1) selon une des revendications 1 à 10, **caractérisée en ce que** dans l'espace du gaz à mesurer (3), en particulier dans l'espace du gaz du corps de sonde (14), un détecteur semi-conducteur spécifique à l'alcool est prévu.
